(19) Europäisches Patentamt / European Patent Office / Office européen des brevets

(11) **EP 4 714 471 A1**

(12) **EUROPEAN PATENT APPLICATION**

(43) Date of publication:
**25.03.2026 Bulletin 2026/13**

(21) Application number: **24306542.2**

(22) Date of filing: **19.09.2024**

(51) International Patent Classification (IPC):
**A61L 2/14** (2006.01)          **H05H 1/00** (2006.01)

(52) Cooperative Patent Classification (CPC):
**A61L 2/14;** A61L 2103/15; A61L 2202/122;
A61L 2202/18; H01J 37/32; H05H 1/4652

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Aurora
76000 Rouen (FR)**
• **Université De Reims Champagne-Ardenne
51100 Reims (FR)**

(72) Inventors:
• **PARIAS, Thomas
76000 Rouen (FR)**
• **LE BRAS, Florian
76000 Rouen (FR)**
• **JUDEÉ, Florian
76000 Rouen (FR)**
• **CHARMOT, Alexandre
76000 Rouen (FR)**
• **VILLEGER, Sandrine
76000 Rouen (FR)**
• **GELLÉ, Marie-Paule
51724 Reims Cedex (FR)**

(74) Representative: **Icosa
83 avenue Denfert-Rochereau
75014 Paris (FR)**

(54) **STERILE ENCLOSURE FOR STERILIZING METHOD AND DEVICE**

(57) The present invention relates to sterilization, and more particularly sterilization of medical devices.

**FIG. 2**

EP 4 714 471 A1

## Description

### FIELD OF INVENTION

The present invention relates to sterilization, and more particularly sterilization of medical devices.

### BACKGROUND OF INVENTION

Sterilizing objects and devices are a crucial aspect for many applications, particularly in medical applications and/or in-vivo applications to avoid bacterial contamination, degradation and/or disease. In the wake of Covid, several sterilizing technologies have been developed, such as UV lights technologies. However, said technologies only shows reduction of bio contaminants levels, but often fail to reach the sterilization as defined in ISO 14937. According to ISO 14937, a process is considered a sterilizing process when, for an object sterilized by the process, the probability of finding contamination by the presence of a biological material (bacteria, viruses, fungus, or spores) on said object is inferior to one out of one million (i.e. a probability lower than $10^{-6}$). For instance, sterilizing an endoscope before an operation on a patient is mandatory, to avoid any risk of complication for the patient. However, sterilization is not an easy straight forward process. The challenge lies in the efficiency of the process against the most resistant strains. Moreover, some objects like endoscopes are complex to sterilize. Indeed, such objects are complex in shape, with difficult parts to reach, and comprise numerous materials, some of which being very sensitive that may present a risk of being damaged by the sterilization process, thus affecting the device function or generating toxic residues.

Among the reference practices, to reach a sterile state, the object to be sterilized may be brought under atmosphere comprising ozone. However, such a method is not able to sterilize organic materials such as cellulose, collagen or other resins and polymers, since it damages said organic material. Furthermore, ozone sterilization is little penetrative, thereby limiting the range of possible applications. Moreover, ozone is generally considered as a dangerous and harmful chemical for human being and therefore its use is banned in a growing number of hospitals. Due to risk of residues, its use for sterilization is not authorized for medical devices. Thus, medical applications are excluded.

Other sterilization methods, compatible with medical applications, are known. In particular, methods involving autoclave or hydrogen peroxide are common for medical applications. However, electronic devices and organic materials are damaged by said methods, which are therefore not recommended for sterilizing them. For instance, heat treatment which are either dry heat at 160°C to 190°C or wet heat at 134°C combined with pressure of 20 to 30 psi (the autoclave method,) have been known and used successfully to sterilize medical devices like metal surgical tools, glass containers and parts. However, with the development use of more complex materials like polymers and electronics, heat treatments reach some limitations: the excess heat may affect the object to be sterilized and either temper with its functionalities or result in premature degradation of polymers with the subsequent loss of mechanical properties, or the generation of harmful or toxic residues, or even deteriorate electronic components or connections. On the other hand, alternative methods requiring less heat which have been developed to address these types of issues generally relies on the use of chemicals like hydrogen peroxide, peracetic acid or ethylene oxide. Nonetheless, chemical methods remain limited in the treatment of complex shape as the chemicals do not penetrate complex shapes like long lumens in endoscopes like gastroscopes and above all may present significant health risk for a patient, because of the residues of the chemical vector which is in use and which are harmful for the body of a patient.

Other sterilization method, involving plasma, are known. However, igniting a plasma requires strict conditions that may not be compatible with the continuity of the sterilized state of an object. For instance, if an object is disposed inside a packaging, plasma may not be able to reach the inside of the packaging and achieve the sterilization of the said object, and on the other hand, if the object is removed from the packaging in order to be directly exposed to the reactive species generated by the plasma to be sterilized, the continuity of the sterile state can not be maintained as it would be recontaminated after the process while being taken by operators to be placed in its packaging.

Also, plasma ignition generally requires a strong energy input resulting in high current and high temperature. Such conditions may affect the devices integrity. This issue has been tentatively addressed by the development of the post discharge plasma treatment. Indeed, this setup suggest placing a plasma source away from the object to be sterilized, then flow the reactive species into a treatment chamber while the active species diffuse around the object to achieve a sterile state. However post-discharge method fails to provide a density of active species into the chamber high enough to preserve the sterile state post treatment. It also fails to achieve a satisfying treatment of complex shapes like long lumens in endoscopes.

Some sterilization technologies based on the use of chemicals such as hydrogen peroxide have been combined with the use of a plasma phase. It allows decomposing the toxic chemicals and reducing residue levels. However, this method still faces the limitations of the diffusion of the chemical species which cannot treat complex shapes.

One of the limiting factors of chemicals and plasma methods is the fact that the object to be sterilized needs to be wrapped in a porous, sterile, barrier to preserve the sterile state after the sterilization process. This barrier is a limitation for two reasons:

- the barrier limits the ability of the chemical to reach the object in an homogenous and sufficient quantity, thus hindering the ability to sterilize complex medical devices such as long lumens like endoscopes.

- the barrier may limit that ability to remove residue from the sterilized device surfaces, and retain chemical residues. Both may be harmful for the further use of the object for patients and operators.

Another aspect, that concerns every sterilization method, is the continuity of the sterilized state. Indeed, the sterilized state is very easily broken when a sterilized object interacts with the environment. More particularly, one may consider that an object loses its sterile state when it interacts with a non-sterilized medium. Said medium may be, for instance, a non-sterilized atmosphere or a non-sterilized object.

However, sterilizing an object with its packaging may hinder the sterilization of said object. Moreover, some packaging may not be able to withstand the conditions imposed by the sterilization methods. One solution may be removing the packaging and sterilizing the object right before its use. However, such a process presents limitations in terms of logistic and infrastructure, even if they are more performant in biological terms since, when a medical device remains contaminated with germs, they quickly develop over time and may form biofilms which are extremely hard to remove. Accordingly, biofilms may lead to expensive replacement of the contaminated parts of the medical device or even lead to the disposal of the entire medical device.

In November 2022, in Barcelona at the annual conference of the World Federation for Hospital Sterilisation Sciences, Dr Lionel Pineau from Eurofins presented a 7 years study on endoscopes contamination in France: Endoscope Reprocessing : Retrospective Analysis of 90311 Samples - in which he reported alarming contamination levels of over 21% of endoscopes in hospitals due to the lack of proper decontamination procedure and especially sterilization. Thus, there is a need for improvement of sterilization methods, in particular for sterilizing complex medical devices.

## SUMMARY

This invention thus relates to a sterile enclosure comprising:

a wall delimiting an inner volume of the sterile enclosure, the wall being permeable to electromagnetic fields and impermeable to biological material,
the wall comprising a barrier portion impermeable to fluid and at least one membrane portion enabling a flow of fluid through said membrane portion,
wherein the barrier portion presents a barrier surface S and the membrane portion comprises an output membrane configured to enable fluid within the inner volume to exit said inner volume, the output membrane presenting an output surface $s_1$, and s1 and S verifies s1/(S+s1) $\leq$0,50 and more preferably 0,010$\leq$ s1/(S+s1) $\leq$0,33.

In the present description, a biological material is any type of living materials. For instance, spores, viruses, bacteria, cells, or fungus, etc... are considered as biological materials.

In the present description, an object is considered sterilized when the probability of presence of a biological material on said object is inferior to $10^{-6}$. According to ISO 14937, it is measured by achieving the complete destruction of $10^6$ colony forming units (cfu) of the most resistant strain- i.e. a 6 logic reduction of a reference strain population.

In the present description, a non-thermal plasma is a gas plasma which is not in thermodynamic equilibrium.

In the present description, a non-thermal plasma is a gas plasma which is not in thermodynamic equilibrium. It is defined with the ionization coefficient

$$\alpha = \frac{n_i}{(n_i + n_n)}$$

where $n_n$ is the density of the gas in which the plasma is active, $n_i$ is the density of positively charged particles and $n_e$ is the electronic density. This coefficient varies from 1 for fully ionized plasma (very hot) down to $10^{-4}$ for low temperature plasma with a low ionization level. Such a low temperature plasma is also called a non-thermal plasma. The following table provides some information on properties of plasma.

| Parameters | Hot Plasma | Non Thermal Plasma |
|---|---|---|
| Temperature | Local Thermodynamic Equilibrium<br>$T_e \cong T_i \cong T_n$<br><br>$\sim 10^4 K \sim 1 eV$ | No Local Thermodynamic Equilibrium<br>$T_e \gg T_i \approx T_n$<br>$T_e \sim 1 - 10 eV$<br>$T_i \sim 300K \sim 1000K$ |
| Density | $n_e \gg 10^{13} cm^{-3}$ | $n_e \approx 10^6 - 10^{13} cm^{-3}$ |
| Ionization Level | High ionization level<br>$\alpha \approx 10^{-2} - 1$ | Partial ionization level<br>$\alpha \approx 10^{-5} - 10^{-2}$ |

In low temperature plasma only a small fraction of the electrons are activated and the fraction of the activated atoms remains small compared to the overall gas atoms typically in the range of $10^6 cm^{-3}$ to $10^{13} cm^{-3}$ more preferably in the range of $3.10^6 cm^{-3}$ to $3.10^8 cm^{-3}$. Hence the overall gas temperature (directly related to the speed of each atoms of molecule in the gas) remains low - here considered lower than 50°C. Non-thermal plasma requires an energy input to ignite and may, for instance, be electrically induced. The non-thermal plasma extinct when the energy input is shut down.

The sterile enclosure of the invention allows keeping a sterilized object inside said sterile enclosure in a sterilized state for at least 30 days most preferably at least 6 months.

Moreover, the sterile enclosure is particularly adapted to function with a sterilizing device using non-thermal plasma. Indeed, since the sterile enclosure allows a flow of a gas, through its membrane, portion inside the inner volume of the sterile enclosure, it is possible to ignite a non-thermal plasma inside the sterile enclosure.

Moreover, the surface ratio allows creating a pressure difference between the inner volume of the sterile enclosure and the exterior. Accordingly, it is possible to ignite a non-thermal plasma in the sterile enclosure in direct contact with the object to be sterilized.

In a further embodiment, the surface ratio allows creating such a pressure difference between the inner volume of the sterile enclosure and the primary enclosure that the non-thermal plasma is selectively ignited inside the sterile enclosure. Thus, it requires less energy to ignite the non-thermal plasma in the sterile enclosure. The sterilization becomes quicker and more reliable.

In some embodiments, the membrane portion further comprises an input membrane, the input membrane presenting an input surface s2, and wherein s1, s2 and S verifies (s1+s2)/(S+s1+s2) ≤0,50 and more preferably 0,010≤ (s1+s2)/(S+s1+s2) ≤0,33 The input membrane allows the sterile enclosure to be connected to a gas supply. Thus, it is easier to generate a gas flow inside the sterile enclosure. Accordingly, it is easier to control the plasma ignition inside the sterile enclosure.

In some embodiments, the enclosure comprises a pipe port configured to convey a gas into the inner volume of the sterile enclosure and configured to be attached to an input pipe, the pipe port comprising an output opening facing the input membrane and a connection part configured to be connected to a gas supply.

The pipe port simplifies the connection of the sterile enclosure to a gas supply.

In some embodiments, the enclosure comprises:

an injection wall attached to the wall and surrounding the input membrane, the injection wall delimiting an input chamber, and
the pipe port protruding into the input chamber, through the injection wall.

The input chamber acts as a decompression chamber, allowing the gas to cross through the membrane portion and fill the inner space of the sterile enclosure without rupture of said enclosure. Moreover, such a configuration allows the injection of a gas inside the inner space of the sterile enclosure without any risk of contamination of said sterile enclosure by biological materials.

In some embodiments, the volume of the input chamber is at least 2 times lower, preferably at least 10 times lower more preferably in range of 100 to 250 times lower, than the inner volume of the sterile enclosure.

Such a configuration improves the reliability of the input chamber acting as a decompression chamber while still insuring a sufficiently high gas injection speed in the sterile enclosure in order to homogenize the renewal of the gas inside the sterile enclosure.

In some embodiments, the input pipe presents an inner diameter in range of 2 to 10 mm.

In some embodiments, the input membrane and output membrane are spaced apart by at least 10 cm along the wall.

Distanced input membrane and output membrane allows the gas flow inside the sterile enclosure to be more homogenous. Accordingly, the sterilization is also more homogenous.

In some embodiments, the membrane portion are made of a porous, non-woven material preferably cellulose or Tyvek©.

Such a material is particularly adapted to act as a membrane, impermeable to biological materials and permeable to a gas flow.

In some embodiments, the barrier portion is made of a polymer material, preferably polyethylene.

Such a material is particularly convenient for endoscopes and for transport of the sterile enclosure, while being cheap and compatible with non-thermal plasma uses.

In some embodiments, the membrane portion is heat sealed on the wall.

Such a configuration is easy to manufacture and is therefore cost efficient.

In some embodiments, the barrier portion comprises a unique opening, configured to open on the inner volume of the sterile enclosure and to be hermetically sealed.

Such an opening is an easy and cheap solution to introduce the object to be sterilized inside the sterile enclosure. In some embodiments, the opening may be reversibly sealed, so the sterile enclosure may be reusable.

In some embodiments, the wall is made of a material having a ductile fractography.

In the present description, the ductile fractography refers to the rupture of a material. In particular, when the material having a ductile fractography is torn apart, it only forms two parts without any crumbling. In other words, the mass of the two obtained parts when the material is torn apart is greater than 99,9%, preferably 99,99%, of the initial mass of said material.

When the sterilized object needs to be retrieved for use, the sterile enclosure is torn apart. However, particularly for medical applications, it is preferable to avoid any solid impurities that may contaminate the sterilized object. Thus, the sterile enclosure presenting a ductile fractography does not generate debris and avoid any contamination.

The present invention also relates to a non-thermal plasma sterilizing device comprising:

a primary enclosure fluidly connected to a pumping system,

the sterile enclosure according to a preceding aspect configured to receive an object to be sterilized, the sterile enclosure being configured to be removably disposed inside the primary enclosure, the output membrane being fluidly connected to the primary enclosure and the secondary enclosure being impermeable to biological materials and permeable to electromagnetic fields,

wherein the sterile enclosure encompasses a gas, the pumping system being configured to pump the gas encompassed in the sterile enclosure through the output membrane, and

wherein the sterilizing device further comprises an electromagnetic source configured to generate an electromagnetic field to ignite the gas of the sterile enclosure into a non-thermal plasma.

Such a sterilizing device advantageously relies on the use of non-thermal plasma in direct contact with the object to be sterilized in order to achieve sterilization. This method is particularly adapted for sensitive material and complex devices. Indeed, non-thermal plasma allows sterilizing all types of material including heat sensitive polymers, resins organic materials such as cellulose, electronics, biomaterials or collagen without concern of time of exposure. The low temperature allows a direct exposition of the complete device to the plasma source where the reactive species are generated.

Moreover, non-thermal plasma lights off as soon as the energy input is turned off. After the plasma extinction the reactive species recombine in a few seconds, leaving no active principle. This is a tremendous advantage of plasma versus other sterilizers: after sterilization, the use of a non-thermal plasma thereby avoids any risks of leaving on the object sterilized any residues that could be harmful for the patient and medical staff health. Accordingly, non-thermal plasma is not only compatible with medical use, but its use is also better than hydrogen peroxide, peracetic acid or ethylene oxide.

## BRIEF DESCRIPTION OF THE DRAWINGS

**Figure 1** is a schema of a non-thermal sterilizing device according to an embodiment of the invention.

**Figure 2** is a schema of a secondary enclosure according to an embodiment of the invention.

**Figure 3** is a graphic showing the evolution of the tension necessary to ignite a gas into a non-thermal plasma versus pressure of said gas.

**Figure 4** is a flow chart showing steps of a method according to one embodiment of the invention.

**DETAILED DESCRIPTION**

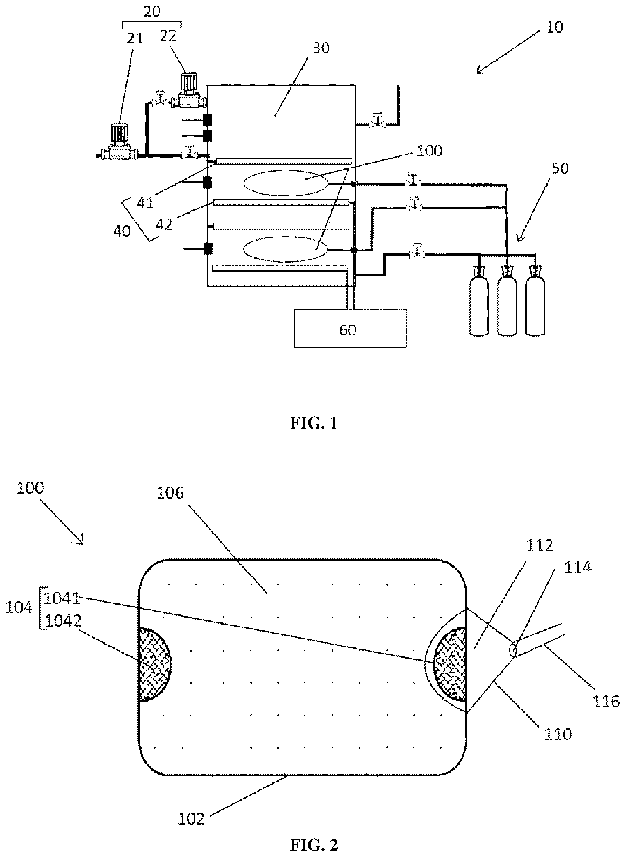

Figure 1 is a schema of a non-thermal sterilizing device 10 according to an embodiment of the invention. The sterilizing device 10 comprises a primary enclosure 30, at least one secondary enclosure 100, a pumping system 20, a gas supply 50, an electromagnetic source 60 and at least one couple of antenna and mass 40 electrically connected to the electromagnetic source 60.

It is noted that the secondary enclosure 100 can also be referred to a sterile enclosure 100. Moreover, in the present description, a sterile enclosure is an enclosure that is impermeable to biological materials. In other words, a sterile enclosure delimits an inner volume that cannot exchange biological materials with anything exterior to said inner volume. The secondary enclosure 100 is a sterile enclosure.

The secondary enclosure 100 is fluidly connected to the gas supply 50 on one hand, and to the primary enclosure 30 on the other hand. The connection to the gas supply 50 is optional. The pumping system 20 comprises a primary pump 21 and a secondary pump 22, each fluidly connected to the primary enclosure 30.

The secondary enclosure 100 is configured to encompass a gas, which may be input by the gas supply 50 or may be present at the beginning within the inner volume of the secondary enclosure 100. The pumping system 20 is configured to pump the gas encompassed inside the secondary enclosure 100. More precisely, the pumping system 20 is configured to pump the gas encompassed in the secondary enclosure 100 indirectly through the vacuum generated in the primary enclosure 30. In other words, the pumping system is configured to generate a vacuum adapted to draw gas from the secondary enclosure 100. When the sterilizing device 10 operates, a gas flow flows from the gas supply 50 to the primary enclosure 30, through the secondary enclosure 100.

The couple of antenna and mass 40 comprises an antenna 42 and a mass 41. The secondary enclosure 100 is disposed between the mass 41 and the antenna 42 of the couple of antenna and mass 40. In the illustrated embodiment, the antenna 42 and the mass 41 are stacked inside the primary enclosure 30 along the vertical direction. In embodiments where the sterilizing device 10 comprises a plurality of couples of antenna and mass 40, the plurality of couples of antenna and mass 40 may be stacked along the vertical direction, inside the primary enclosure 30. Other stacking may be considered, such as horizontal stacking.

The sterilizing device 10 may comprise coils disposed around the primary enclosure 30. The coils are configured to generate a magnetic field inside the secondary enclosure 100. The coils may be electrically connected to the electromagnetic source 60 or be connected to an independent power source.

The electromagnetic source 60 may be a standard power source, optionally connected to the standard main power. The electromagnetic source 60 may provide power to other equipment of the sterilizing device, such as display devices.

Figure 2 is a detailed schema of the secondary enclosure 100 according to an embodiment of the invention. The secondary enclosure 100 comprises a wall 102 delimiting the inner volume of the secondary enclosure 100. The wall 102 comprises a membrane portion 104 and a barrier portion 106. The wall 102 is permeable to electromagnetic fields and impermeable to biological material. Moreover, the membrane portion 104 enables flow of a fluid, for instance a gas, through said membrane portion 104 while the barrier portion 106 is impermeable to said fluid.

The wall 102 of the secondary enclosure 100 may be flexible or rigid. Is considered flexible a material that presents a Young's modulus E is typically in the range of 0,1 to 5Gpa.

$$E = \frac{\varepsilon}{\sigma} = \frac{\text{Strain}}{\text{Stress}}$$

Where:

- Stress ($\sigma$) is the force applied per unit area (e.g., in Pascals, Pa).
- Strain ($\varepsilon$) is the relative deformation, or change in length, compared to the original length (dimensionless).

A material that is not flexible is considered rigid. For instance, the barrier portion 106 of the wall 102 may be a polymer such as polyethylene, polypropylene or PVC. The membrane portion 104 may be a porous typically a non-woven material. Such a material may comprise pores sizing from 10 to 300 nm, so as to prevent biological material to pass through this material. More concretely, the Log Reduction Value [LRV] of the material is greater than 5 according to ASTM F1608. The porosity will allow a permeability to gas flowthrough which could be characterized according to ISO 5636-5 by the Berdtsen Air Permeability Method and would be typically in the range of 0.3 to 3 kPa/10 cm$^3$. For instance, the membrane portion 104 may be Tyvek $^©$ or cellulose.

The membrane portion 104 is attached to the barrier portion 106. For instance, the membrane portion 104 may be heat sealed, ultrasound sealed, glued or mechanically sealed, for instance with clipses on the barrier portion 106.

Advantageously, the barrier portion 106 presents a ductile fractography. In other words, when the barrier portion 106 is torn apart, it only forms two parts without any crumbling. More particularly, the mass of the two obtained parts when the barrier portion 106 is torn apart is greater than 99,9%, preferably 99,99%, of the initial mass of the barrier portion 106.

The membrane portion 104 comprises an input membrane 1041 and an output membrane 1042. The input membrane 1041 and the output membrane 1042 are spaced apart by at least 10 cm along the wall 102. More generally, the input membrane 1041 and the output membrane 1042 may be spaced apart by at least 33% more preferably between 33% to 66% of the longest dimension of the wall 102.

The secondary enclosure 100 further comprises an injection wall 110 attached to the wall 102. The injection wall 110 surrounds the input membrane 1041 and delimits an input chamber 112. A pipe port 114 is provided on the injection wall 110 and is configured to be attached to an input pipe 116, the pipe port 114 being configured to allow a flow of gas to pass through itself, into the input chamber 112. On the other hand, the input pipe 116 is connected to the gas supply 50.

The secondary enclosure 100 comprises an opening that opens in the inner volume of the secondary enclosure 100. The secondary enclosure 100 is configured to receive an object to be sterilized inside its inner volume. The object may be put inside the secondary enclosure 100 through its opening. Once the object is placed inside the secondary enclosure 100, the opening is sealed. The sealing may be a heat seal, glueing, ultrasound sealed, mechanically sealed or any type of sealing that prevents biological material to pass through.

The gas supply 50 is configured to deliver a gas to the input chamber 112 through the input pipe 116. The gas then passes through the input membrane 1041, into the inner volume of the secondary enclosure. The permeability of the input membrane 1041 generates a pressure difference between the input chamber 112 and the inner volume of the secondary enclosure 100. When the pressure in the secondary enclosure 100 reaches below $10^{-3}$ bars enclosure, the pressure of the primary enclosure 30 is lower than 10 times, most preferably in the range of 100 to 1000 times than the pressure inside the secondary enclosure 100. Accordingly, at such a time, the pressure inside the primary enclosure 30 is below $9.10^{-4}$ bars.

In some embodiments, the secondary enclosure 100 may comprise only a pipe port 114, directly attached to the input membrane 1041.

When a gas flows through the secondary enclosure 100, a pressure difference appears between the primary enclosure 30 and the secondary enclosure 100. This pressure difference can be estimated by Darcy's law as shown in equation (1).

$$\frac{Q}{A} = \frac{k(P_2 - P_1)}{\eta.D_x} \quad : \quad (1)$$

In equation (1), Q is the gas input flow rate, A is the surface of the membrane portion 104, k is the permeability to the gas of the membrane portion 104, $P_2$ is the pressure inside the secondary enclosure 100, Pi is the pressure inside the primary enclosure 30, $\eta$ is the viscosity of the gas and $D_x$ is the thickness of the membrane portion 104.

Accordingly, it is possible to adapt the pressure difference between the primary enclosure 30 and the secondary enclosure 100 by adapting the area of the membrane portion 104 of the secondary enclosure 100.

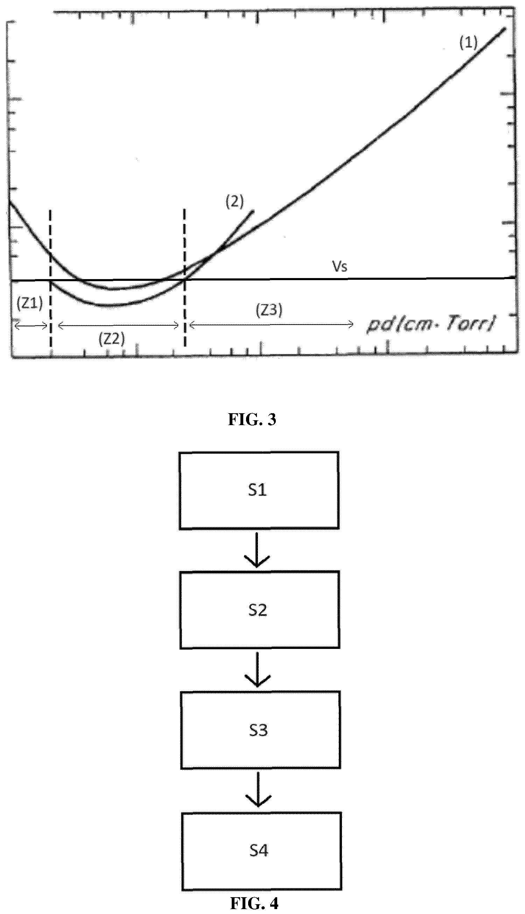

Figure 3 is a graphic showing the evolution of the tension necessary to ignite a gas into a non-thermal plasma versus pressure of said gas. These data are Paschen curves. The ignition tension has been measured for two different gases: air (1) and nitrogen (2).

As shown on figure 3, the required voltage for igniting a plasma presents a minimum. In other words, when the gas pressure is either low or high, an important voltage is required for igniting a plasma. For instance, if a tension Vs, greater than the minimal tension for igniting a plasma, is applied to a gas, three pressure zones are delimited, function of said gas pressure.

In the first zone Z1, which is the low-pressure zone, the pressure of the gas is too low for the tension Vs to ignite the plasma. In the second zone Z2, the pressure of the gas allows the plasma ignition. In the third zone Z3, the pressure of the gas is too high for the tension Vs to ignite the plasma.

In the present invention, the electromagnetic source generates a target tension Vs between the antenna 42 and the mass 41. Since the secondary enclosure 100 is disposed inside the primary enclosure 30, between the antenna 42 and the mass 41, the target tension Vs is applied to the gas encompassed inside the secondary enclosure 100. Moreover, the target tension Vs is also applied to the gas encompassed inside the primary enclosure 30, which is between the antenna 42 and the mass 41.

Accordingly, the difference in pressure between the secondary enclosure 100 and the primary enclosure 30 is adjusted so the pressure inside the primary enclosure 30 is too low for a plasma to be ignited while the pressure inside the secondary enclosure 100 allows the plasma ignition. In other words, referring to figure 3, the gas inside the primary enclosure 30 is in zone Z1 while the gas encompassed inside the secondary enclosure 100 is in zone Z2. Thus, the non-thermal plasma can be selectively ignited inside the secondary enclosure 100.

Preferably, the gas supply 50 provides air and the gas pressure inside the secondary enclosure 100 is below $10^{-2}$

bars more preferably in range of $10^{-3}$ to $10^{-5}$ bars. On the other hand, the gas pressure inside the primary enclosure 30 is below $9.10^{-4}$ bars more preferably in range of $10^{-7}$ to $10^{-5}$ bars. The gas supply may provide nitrogen, oxygen, carbon dioxide, air or argon or mixture thereof.

As explained above the pressure difference between the secondary enclosure 100 and the primary enclosure 30 is proportional to the surface of the membrane portion 104 (see equation (1)). The output membrane 1042 presents an output surface s1 and the input membrane 1041 presents an input surface $s_2$. Accordingly, the surface of the membrane portion 104 is $s_1+s_2$. On the other hand, the barrier portion 106 presents a surface S. Accordingly, the total surface of the secondary enclosure 100 is $s_1+s_2+S$.

The surfaces of the membrane portion 104 and the barrier portion 106 of the secondary enclosure 100 are configured to have the ratio $\Gamma$ (2) equal or lower than 50% more preferably between 33% and 1,0%.

$$\Gamma = \frac{s_1 + s_2}{S + s_1 + s_2} : (2)$$

When the surfaces of the secondary enclosure 100 verify ratio $\Gamma$ (2), the gas pressure inside the secondary enclosure 100 is, according to equation (1), at least 10 times and preferably in range of 100 to 1000 times higher than the gas pressure inside the primary enclosure 30 when the pressure enclosure is established below $9.10^{-4}$ bars.

Figure 4 is a flow chart showing steps of a method according to one embodiment of the invention. The method comprises:

- Placing an object to sterilize inside the secondary enclosure 100,

- Placing the secondary enclosure 100 into the primary enclosure 30 of the sterilizing device 10,

- Igniting a plasma inside the secondary enclosure 100,

- Collecting the secondary enclosure 100 containing the sterilized object.

In step S1, the object to be sterilized is placed inside the secondary enclosure 100, through the opening of said secondary enclosure 100. Any object may be placed inside the secondary enclosure 100. However, the secondary enclosure 100 is particularly adapted to medical devices such as endoscopes. Moreover, the opening of the secondary enclosure 100 is sealed, while the secondary enclosure 100 encompasses the object to be sterilized. In addition, a gas may also be encompassed inside the secondary enclosure.

In step S2, the secondary enclosure 100 encompassing the object is placed inside the sterilizing device 10, inside the primary enclosure 30, between the couple of antenna and mass 40. The pipe port 114 of the secondary enclosure is connected to the gas supply 50 through the pipe 116.

Then, the primary enclosure 30 is closed and the pumping system 20 pumps the gas inside the primary enclosure up to a target pressure, below $9.10^{-4}$ bars, more preferably in range of $10^{-7}$ bar to $10^{-6}$ bar. On the other hand, the gas supply 50 is opened to create an airflow inside the secondary enclosure 100. The pressure in the secondary enclosure 100 is maintained in range of $10^{-5}$ bar to $10^{-4}$ bar.

In step S3, the electromagnetic source 60 is turned on, such that an electromagnetic field is generated between the antenna 42 and the mass 41 of the couple of antenna and mass 40. In particular, a target tension Vs is set between the antenna 42 and the mass 41. The target tension Vs is sufficient to ignite a non-thermal plasma inside the secondary enclosure 100 without igniting any plasma inside the primary enclosure 30.

The electromagnetic source 60 is configured to generate a pulsatile or discontinuous electromagnetic field comprising an active phase and a shutoff phase. During the active phase, the electromagnetic field sets the target tension Vs between the antenna 42 and the mass 41. During the shutoff phase, the electromagnetic field is shutdown. One active phase followed by one shutoff phase is a cycle. During step S4, at least 3 cycles are performed more preferably 10 to 15 cycles.

The active phase lasts for less than 5 minutes, preferably less than 2 minutes. The shutdown phase lasts for less than 5 minutes, preferably less than 1 minute. Accordingly, the frequency of the pulsatile electromagnetic field is in range of 10 to 100 MHz more preferably 13 to 15MHz. At the end of step S4, the object disposed inside the secondary enclosure 100 is sterilized.

In step S4, the secondary enclosure 100 is removed from the sterilizing device 10, and may be stored sealed and/or transported for a further use.

**Claims**

1. Sterile enclosure (100) comprising:

   a wall (102) delimiting an inner volume of the sterile enclosure (100), the wall (102) being permeable to electromagnetic fields and impermeable to biological material,
   the wall (102) comprising a barrier portion (106) impermeable to fluid and at least one membrane portion (104) enabling a flow of fluid through said membrane portion (104),
   wherein the barrier portion (106) presents a barrier surface (S) and the membrane portion (104) comprises an output membrane (1042) configured to enable fluid within the inner volume to exit said inner volume, the output membrane (1042) presenting an output surface s 1, and s1 and S verifies $s1/(S+s1) \leq 0,50$ and more preferably $0,010 \leq s1/(S+s1) \leq 0,33$.

2. The sterile enclosure (100) of claim 1, wherein the membrane portion (104) further comprises an input membrane (1041), the input membrane (1041) presenting an input surface s2, and wherein s1, s2 and S verifies $(s1+s2)/(S+s1+s2) \leq 0,50$ and more preferably $0,010 \leq (s1+s2)/(S+s1+s2) \leq 0,33$.

3. The sterile enclosure (100) of claim 2, further comprising a pipe port (114) configured to convey a gas into the inner volume of the sterile enclosure (100) and configured to be attached to an input pipe (116), the pipe port (114) comprising an output opening facing the input membrane (1041) and a connection part configured to be connected to a gas supply.

4. The sterile enclosure (100) of claim 3, further comprising:

   an injection wall (110) attached to the wall and surrounding the input membrane (1041), the injection wall (110) delimiting an input chamber (112), and
   the pipe port (114) protruding into the input chamber (112), through the injection wall (110).

5. The sterile enclosure (100) of claim 4, wherein the volume of the input chamber (112) is 2 times lower, preferably in range of 100 to 250 times lower, than the inner volume of the sterile enclosure (100).

6. The sterile enclosure (100) of any of claims 3 to 5, wherein the input pipe (116) presents an inner diameter in range of 2 to 10 mm.

7. The sterile enclosure (100) of any of claims 2 to 6, wherein the input membrane (1041) and output membrane (1042) are spaced apart by at least 10 cm along the wall.

8. The sterile enclosure (100) of any of claim 1 to 7, wherein the membrane portion (104) is made of a porous, non-woven material preferably cellulose or Tyvek©.

9. The sterile enclosure (100) of any of claim 1 to 8, wherein the barrier portion (106) is made of a polymer material, preferably polyethylene.

10. The sterile enclosure (100) of any of claims 1 to 9, wherein the membrane portion (104) is heat sealed on the wall.

11. The sterile enclosure (100) of any of claims 1 to 10, wherein the barrier portion (106) comprises a unique opening, configured to open on the inner volume of the sterile enclosure (100) and to be hermetically sealed.

12. The sterile enclosure (100) of any of claims 1 to 11, wherein the wall is made of a material having a ductile fractography.

13. A non-thermal plasma sterilizing (10) device comprising:

   a primary enclosure (30) fluidly connected to a pumping system (20),
   the sterile enclosure (100) according to claims 1 to 13 configured to receive an object to be sterilized, the sterile enclosure (100) being configured to be removably disposed inside the primary enclosure (30), the output membrane (1041) being fluidly connected to the primary enclosure (30) and, the sterile enclosure (100) being impermeable to biological material and permeable to electromagnetic field,
   wherein the sterile enclosure (100) encompasses a gas, the pumping system (20) being configured to pump the gas encompassed in the sterile enclosure (100) through the output membrane, and
wherein the sterilizing device further comprises an electromagnetic source (60) configured to generate an electromagnetic field to ignite the gas of the sterile enclosure (100) into a non-thermal plasma.

**FIG. 1**

**FIG. 2**

**FIG. 3**

**FIG. 4**

Europäisches
Patentamt

European
Patent Office

Office européen
des brevets

**EUROPEAN SEARCH REPORT**

**Application Number**

EP 24 30 6542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/230426 A1 (POPOT JEAN-MARC [FR] ET AL) 5 September 2013 (2013-09-05)<br>* paragraph:[0056], [0057], [0059], [0060], [0066], [0070], [0071], [0072], [0074], [0076];<br>figure: 1 *<br>----- | 1-13 | INV.<br>A61L2/14<br>H05H1/00 |
| X | WO 2019/074886 A1 (TERUMO BCT BIOTECHNOLOGIES LLC [US])<br>18 April 2019 (2019-04-18)<br>* paragraph: [0028], [0043], [0044], [0063];<br>claim: 19;<br>figure: 1A *<br>----- | 1,8-10, 12 | |

|  |
|---|
| TECHNICAL FIELDS SEARCHED (IPC) |
| A61L<br>H05H |

The present search report has been drawn up for all claims

| Place of search | Date of completion of the search | Examiner |
|---|---|---|
| Munich | 13 February 2025 | Nania, Manuela |

# EP 4 714 471 A1

## ANNEX TO THE EUROPEAN SEARCH REPORT
## ON EUROPEAN PATENT APPLICATION NO.

EP 24 30 6542

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2025

| Patent document cited in search report | Publication date | Patent family member(s) | Publication date |
|---|---|---|---|
| US 2013230426 A1 | 05-09-2013 | CA 2819374 A1 | 29-03-2012 |
| | | EP 2618851 A1 | 31-07-2013 |
| | | ES 2515491 T3 | 29-10-2014 |
| | | FR 2965179 A1 | 30-03-2012 |
| | | US 2013230426 A1 | 05-09-2013 |
| | | WO 2012038669 A1 | 29-03-2012 |
| WO 2019074886 A1 | 18-04-2019 | CA 3078625 A1 | 18-04-2019 |
| | | CN 111295094 A | 16-06-2020 |
| | | EP 3694322 A1 | 19-08-2020 |
| | | JP 7110360 B2 | 01-08-2022 |
| | | JP 7227417 B2 | 21-02-2023 |
| | | JP 2021500919 A | 14-01-2021 |
| | | JP 2022119803 A | 17-08-2022 |
| | | US 2019106254 A1 | 11-04-2019 |
| | | US 2021016943 A1 | 21-01-2021 |
| | | WO 2019074886 A1 | 18-04-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DR LIONEL PINEAU**. Endoscope Reprocessing : Retrospective Analysis of 90311 Samples. *Barcelona at the annual conference of the World Federation for Hospital Sterilisation Sciences*, November 2022